# EUROPEAN PATENT SPECIFICATION

(11) **EP 3 751 516 B1**
(45) Date of publication and mention of the grant of the patent: **28.06.2023**
(21) Application number: 19179411.4
(22) Date of filing: 11.06.2019
(51) Int. Cl.: G06T 7/11

(54) **AUTONOMOUS MULTIDIMENSIONAL SEGMENTATION OF ANATOMICAL STRUCTURES ON THREE-DIMENSIONAL MEDICAL IMAGING**
AUTONOME MEHRDIMENSIONALE SEGMENTIERUNG ANATOMISCHER STRUKTUREN AUF DREIDIMENSIONALER MEDIZINISCHER BILDGEBUNG
SEGMENTATION MULTIDIMENSIONNELLE AUTONOME DE STRUCTURES ANATOMIQUES SUR UNE IMAGERIE MÉDICALE TRIDIMENSIONNELLE

(43) Date of publication of application: 16.12.2020
(73) Proprietor: Holo Surgical Inc., Deerfield, IL 60015 (US)
(72) Inventor: Siemionow, Krzysztof B., Chicago, IL Illinois 60610 (US); Luciano, Cristian J., Evergreen Park, IL Illinois 60805 (US); Gawel, Dominik, 01-949 Warszawa (PL); Trzmiel, Michal, 01-949 Warszawa (PL); Mejia Orozco, Edwing Isaac, 01-949 Warszawa (PL)
(74) Representative: Kancelaria Eupatent.pl Sp. z.o.o

(56) References cited:
- EP-A1- 3 432 263
- WO-A1-2019/005722
- WO-A2-2007/110820
- US-A1- 2019 130 575

## Description

### TECHNICAL FIELD

The present disclosure generally relates to multidimensional autonomous segmentation of anatomical structures on three dimensional (3D) medical imaging, useful in particular for the field of computer assisted surgery, diagnostics, and surgical planning.

### BACKGROUND

Image guided or computer assisted surgery is a surgical procedure where the surgeon uses tracked surgical instruments in conjunction with preoperative or intraoperative images in order to indirectly guide the procedure. Image guided surgery can utilize images acquired intraoperatively, provided for example from computer tomography (CT) scanners.

Specialized computer systems can be used to process the CT images to develop three-dimensional models of the anatomy fragment subject to the surgery procedure.

For this purpose, various machine learning technologies are developed, such as a convolutional neural network (CNN) that is a class of deep, feed-forward artificial neural networks. CNNs use a variation of feature detectors and/or multilayer perceptrons designed to require minimal preprocessing of input data.

In the field of image guided surgery, low quality images may make it difficult to adequately identify key anatomic landmarks, which may in turn lead to decreased accuracy and efficacy of the navigated tools and implants. Furthermore, low quality image datasets may be difficult to use in machine learning applications.

WO2007110820 discloses a process for automatic recognition of abnormalities in anatomical structures, comprising the operations of: acquiring a plurality of two-dimensional images of at least one portion of a patient's body capable of forming a three-dimensional representation of at least one anatomical structure under observation, segmenting a region of interest in the said three-dimensional representation which potentially contains anomalies, selecting volume image elements (voxels) from the segmented region which are likely to form a part of abnormalities in the anatomical structure represented, on the basis of predetermined morphological parameters, agglomerating the said voxels into analysis units on the basis of the distribution of the spatial density of the voxels selected, and classifying the said analysis units as elements suspected of being parts of abnormalities or as elements which are not part of abnormalities.

Computer Tomography (CT) is a common method for generating a three-dimensional (3D) image of the patient's anatomy. CT scanning works like other x-ray examinations. Very small, controlled amounts of x-ray radiation are passed through the body, and different tissues absorb radiation at different rates. With plain radiology, when special film is exposed to the absorbed x-rays, an image of the inside of the body is captured. With CT, the film is replaced by an array of detectors, which measure the x-ray profile.

The CT scanner contains a rotating gantry that has an x-ray tube mounted on one side and an arc-shaped detector mounted on the opposite side. An x-ray beam is emitted in a fan shape as the rotating frame spins the x-ray tube and detector around the patient. Each time the x-ray tube and detector make a 360° rotation and the x-ray passes through the patient's body, the image of a thin section is acquired. During each rotation, the detector records about 1,000 images (profiles) of the expanded x-ray beam. Each profile is then reconstructed by a dedicated computer into a 3-dimensional image of the section that was scanned. The speed of gantry rotation, along with slice thickness, contributes to the accuracy/usefulness of the final image.

Commonly used intraoperative scanners have a variety of settings that allow for control of radiation dose. In certain scenarios high dose settings may be chosen to ensure adequate visualization of all the anatomical structures. The downside of this approach is increased radiation exposure to the patient. The effective doses from diagnostic CT procedures are typically estimated to be in the range of 1 to 10 mSv (millisieverts). This range is not much less than the lowest doses of 5 to 20 mSv estimated to have been received by survivors of the atomic bombs. These survivors, who are estimated to have experienced doses slightly larger than those encountered in CT, have demonstrated a small but increased radiation-related excess relative risk for cancer mortality.

The risk of developing cancer as a result of exposure to radiation depends on the part of the body exposed, the individual's age at exposure, and the individual's gender. For the purpose of radiation protection, a conservative approach that is generally used is to assume that the risk for adverse health effects from cancer is proportional to the amount of radiation dose absorbed and that there is no amount of radiation that is completely without risk.

Low dose settings should be therefore selected for computer tomography scans whenever possible to minimize radiation exposure and associated risk of cancer development. However, low dose settings may have an impact on the quality of the final image available for the surgeon. This, in turn, can limit the value of the scan in diagnosis and treatment.

Magnetic resonance imaging (MRI) scanner forms a strong magnetic field around the area to be imaged. In most medical applications, protons (hydrogen atoms) in tissues containing water molecules create a signal that is processed to form an image of the body. First, energy from an oscillating magnetic field is applied temporarily to the patient at the appropriate resonance frequency. The excited hydrogen atoms emit a radio frequency signal, which is measured by a receiving coil. The radio signal may be made to encode position information by varying the main magnetic field using gradient coils. As these coils are rapidly switched on and off they create the characteristic repetitive noise of an MRI scan. The contrast between different tissues is determined by the rate at which excited atoms return to the equilibrium state. Exogenous contrast agents may be given intravenously, orally, or intra-articularly.

The major components of an MRI scanner are: the main magnet, which polarizes the sample, the shim coils for correcting inhomogeneities in the main magnetic field, the gradient system which is used to localize the MR signal and the RF system, which excites the sample and detects the resulting NMR signal. The whole system is controlled by one or more computers.

The most common MRI strengths are 0.3T, 1.5T and 3T, where "T" stands for Tesla - the unit of measurement for the strength of the magnetic field. The higher the number, the stronger the magnet. The stronger the magnet, the higher the image quality. For example, a 0.3T magnet strength will result in lower quality imaging than a 1.5T. Low quality images may pose a diagnostic challenge, as it may be difficult to identify key anatomical structures or a pathologic process. Low quality images also make it difficult to use the data during computer assisted surgery. Therefore, it is important to have the ability to deliver a high-quality MRI images for the physician.

### SUMMARY OF THE INVENTION

There is disclosed herein a method and a computer-implemented system for autonomous multidimensional segmentation of anatomical structures from three-dimensional (3D) scan volumes according to the appended claims.

### BRIEF DESCRIPTION OF DRAWINGS

The invention is shown by means of example embodiments on drawings, wherein:
Fig. 1 shows a neural network training procedure;
Figs. 2A-2C show exemplary, single 2D images from exemplary 3D volume sets used in the system during the procedures;
Figs. 2D-1 and 2D-2 show exemplary, automatically defined multidimensional regions used in the process;
Figs. 2E-1 and 2E-2 show three-dimensional resizing of exemplary region;
Fig. 2F shows exemplary transformations for data augmentation;
Fig. 3 shows an overview of an autonomous multidimensional segmentation procedure;
Fig. 4 shows a general CNN architecture used for multidimensional segmentation of anatomical structures;
Fig. 5 shows a flowchart of a training process of the CNN for the multidimensional segmentation of anatomical structures;
Fig. 6 shows a flowchart of CNN inference process for multidimensional segmentation of anatomical structures;
Fig. 7 shows exemplary results of filtering autonomous multidimensional segmentation results;
Fig. 8 shows a computer-implemented system for implementing the segmentation procedure.

### DETAILED DESCRIPTION

The invention relates to processing three-dimensional scan volume comprising a set of medical scan images of the anatomical structures including, but not limited to, vessels (aorta and vena cava), nerves (cervical, thoracic or lumbar plexus, spinal cord and others), bones, and widely defined soft and hard tissues. The invention will be presented below based on an example of vascular anatomical structures comprising the aorta and vena cava in the neighborhood of a spine as a bone structure, but the method and system can be equally well used for any other three-dimensional anatomical structures visible on medical imaging.

Moreover, the present invention may include, before segmentation, pre-processing of low-quality images to improve the visibility of different tissues. This can be done by employing a method presented in a European patent application EP16195826 by the present applicant or any other pre-processing quality improvement method. The low-quality images may be, for example, low dose computed tomography (LDCT) images or magnetic resonance images captured with a relatively low power scanner.

The foregoing description will present examples related to computed tomography (CT) images, but a skilled person will realize how to adapt the embodiments to be applicable to other image types, such as magnetic resonance imaging (MRI).

The multidimensional segmentation of anatomical structures method, as presented herein, comprises two main procedures: human-assisted, supervised (manual) training, and autonomous segmentation. The word "multidimensional" is used herein to define a dimensionality equal or higher than three. The number of dimensions depends on the amount of information obtained from convergent sources.

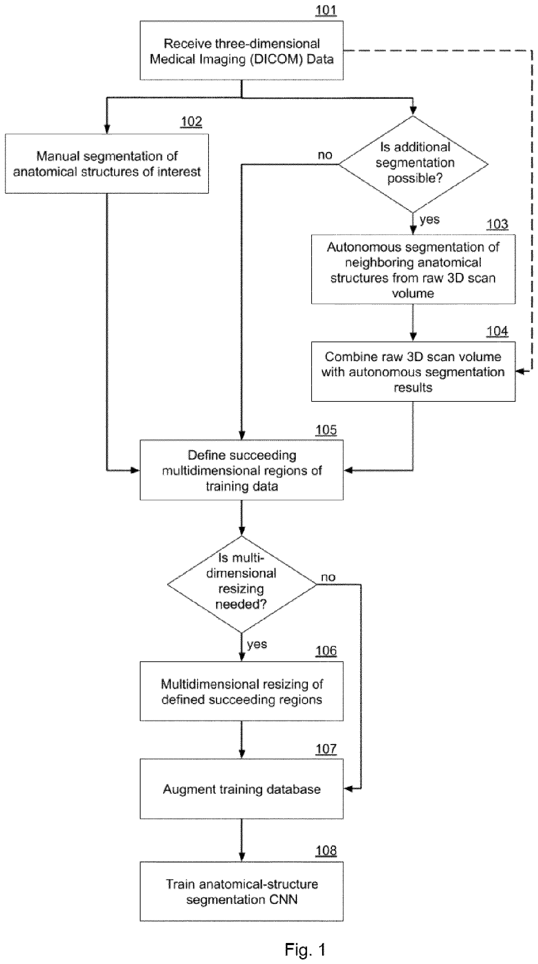

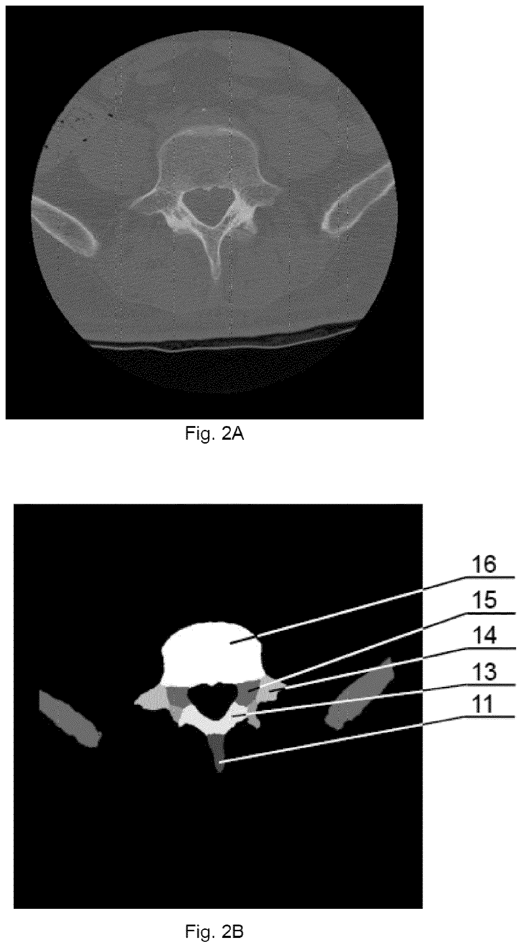

The training procedure, as presented in Fig. 1, comprises the following steps. Firstly, in step 101, a set of DICOM (Digital Imaging and Communications in Medicine) images obtained from a preoperative or intraoperative CT or MRI scanner, representing consecutive slices of the anatomical structures (as shown in Fig. 2A) is received in a form of a 3D scan volume.

Next, in step 102, the anatomical structures of interest are manually marked by a human on the raw 3D scan volume, to prepare an initial training database, comprising raw, three-dimensional DICOM as an input and manually marked, color-coded representation of the anatomical structures corresponding to the input data.

If possible, the raw 3D scan volume is processed in step 103 to perform initial autonomous segmentation of the neighboring tissues, in order to determine separate areas corresponding to the well seen structures (for example bony structure, and its parts such as vertebral body 16, pedicles 15, transverse processes 14, lamina 13 and/or spinous process 11, as shown in Fig. 2B). This can be done by employing the present invention, a method for segmentation of images disclosed in a European patent application EP16195826 by the present applicant, or any other segmentation method, that provides as an output representation of anatomical parts.

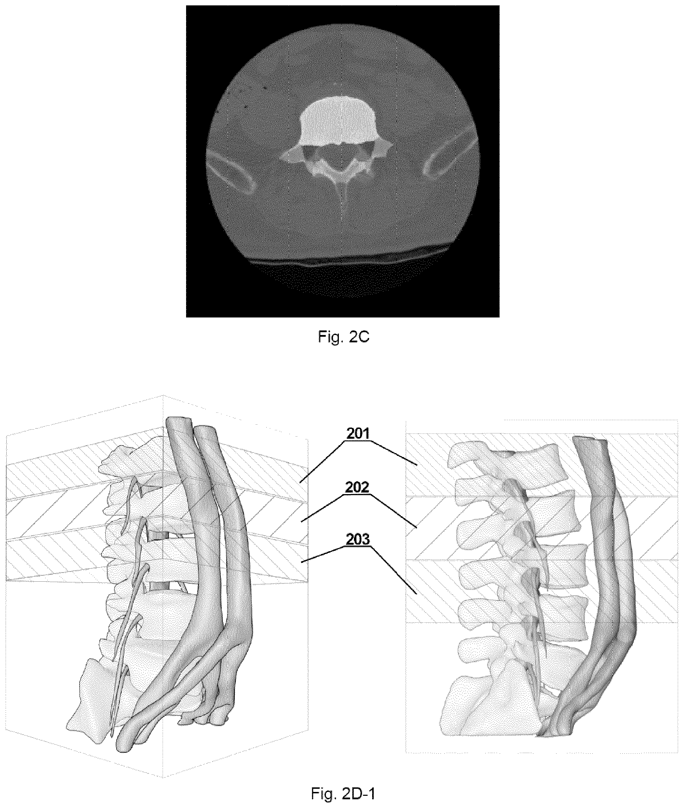

Then, if step 103 is performed, the raw information from 3D scan volume and the autonomous segmentation results (from step 103) are merged in step 104. Combining the information about appearance and classification of neighboring anatomical structures increases the amount of information used for the network inference in further autonomous segmentation process by increasing the dimensionality of the input data. This can be achieved, for example, by modifying the input data to take the form of color-coded 3D volumes, as shown in Fig. 2C. Alternatively, the process may take place directly inside of the neural network, where the separately introduced 3D scan volumes (Fig. 2A) and the initial segmentation results (Fig. 2B) can be passed to a neural network inputs and automatically concatenated, to produce the processed information of higher dimensionality.

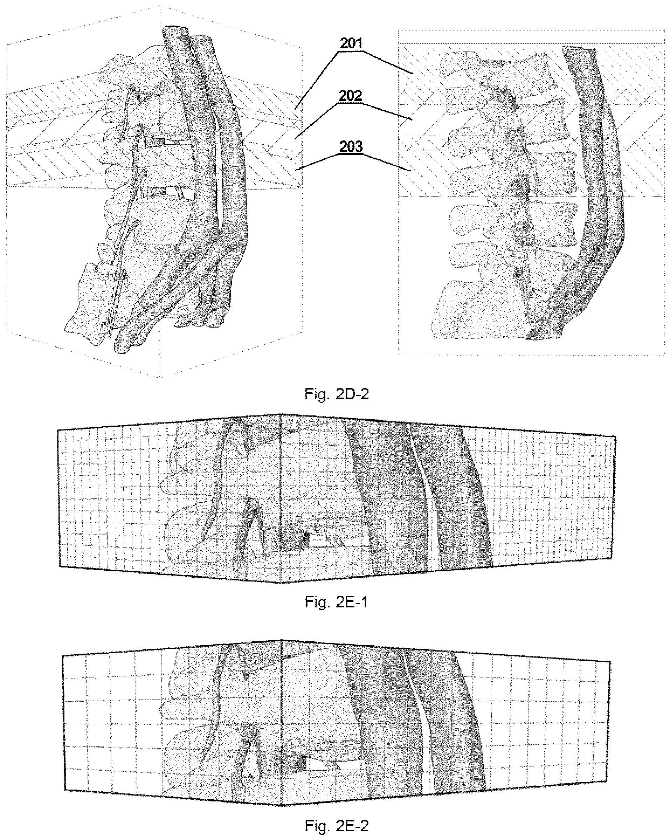

Next, in step 105, succeeding multidimensional regions of training data (for example 201, 202, and 203) are determined using predefined parameters, such as the size of the region or the multidimensional stride. An example of regions separated by a stride equal to one dimension of the region is shown on Fig. 2D-1, and with a smaller stride that allow overlapping of regions is shown on Fig. 2D-2. The neural network training comprises information from the raw 3D scan volumes (or the merged information from step 104) and manual segmentation results (from step 102).

Then, in step 106, if requested, the automatically defined (105) succeeding multidimensional regions are being subjected to multidimensional resizing, to achieve the predefined size (Fig. 2E-1 and Fig. 2E-2).

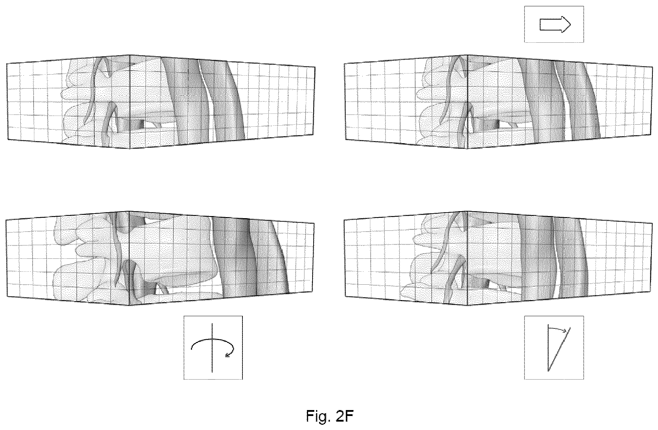

Next, in step 107, the training database is augmented, as shown in Fig. 2F. Data augmentation is performed in order to make the training set more diverse. The input/output multidimensional regions pairs are subjected to the same combination of transformations from the following set: rotation, translation, scaling, shear, horizontal or vertical flip, multidimensional grid deformations, additive noise of Gaussian and/or Poisson distribution and Gaussian blur, brightness or contrast corrections, etc. The aforementioned multidimensional generic geometrical transformations with dense multidimensional grid deformations remap the voxels positions in multidimensional regions based on a randomly warped artificial grid assigned to the volume. A new set of voxel positions is calculated artificially warping the anatomical structures shape and appearance. Simultaneously, the information about the anatomical structures' classification is warped to match the new anatomical structures' shape and the manually indicated anatomical structures are recalculated in the same manner. During the process, the value of each voxel, containing information about the anatomical structures' appearance, is recalculated in regard to its new position using an interpolation algorithm (for example: bicubic, polynomial, spline, nearest neighbor, or any other interpolation algorithm) over the voxel neighborhood.

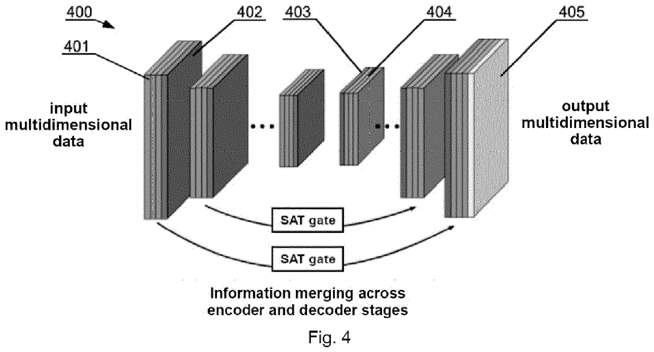

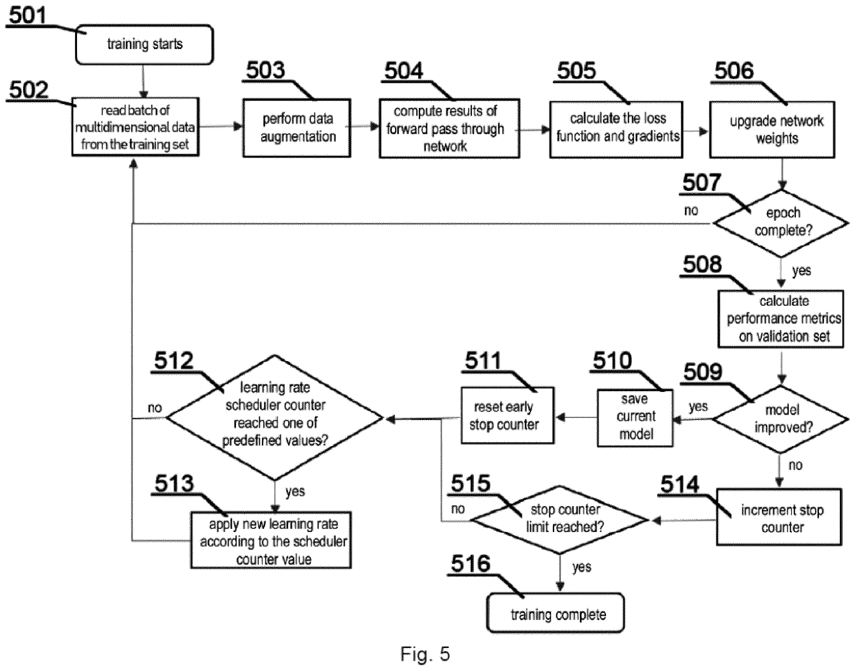

Then, in step 108, a convolutional neural network (CNN) is trained with training data comprising information from the raw 3D scan volumes (or the merged information from step 104) and manual segmentation results (from step 102). For example, a network such as shown in Fig. 4 can be trained according to the network training procedure, as shown in Fig. 5. Additionally Select-Attend-Transfer (SAT) gates or Generative Adversarial Networks (GAN) can be used to increase the final quality of the segmentation.

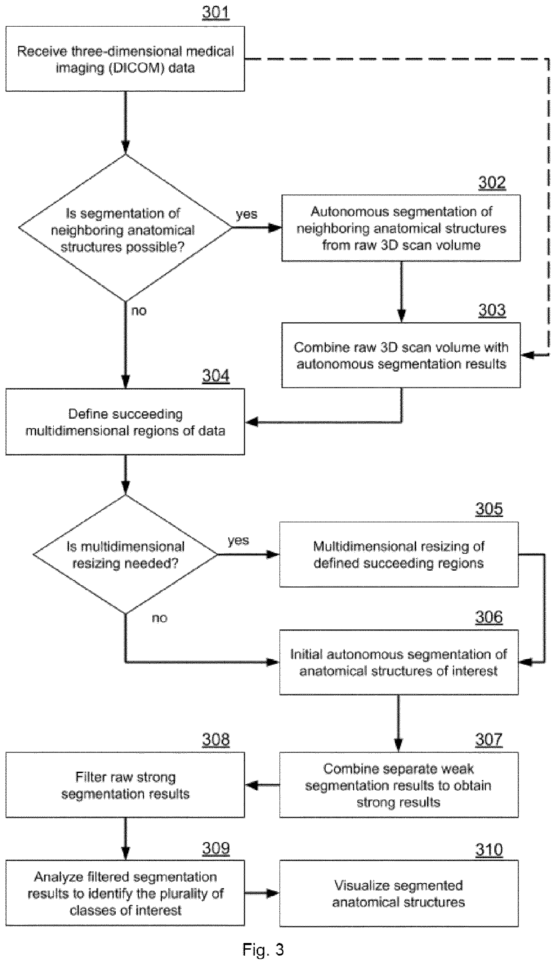

The autonomous segmentation procedure for multidimensional anatomical structures, as presented in Fig. 3, comprises the following steps. First, in step 301, a raw 3D scan volume is received, comprising a set of DICOM images presenting a volumetric region with anatomical structures or its part. The raw 3D scan volume can be obtained from a preoperative or intraoperative CT or MRI.

Next, if possible, the raw 3D scan volume is processed in step 302 to perform autonomous segmentation of well recognizable neighboring anatomical structures, for example spine and its parts, such as: vertebral body 16, pedicles 15, transverse processes 14, lamina 13 and/or spinous process 11, as shown in Fig. 2B - thereafter called autonomous segmentation results. This can be done by employing the present invention, a method for segmentation of images disclosed in a European patent application EP16195826 by the present applicant, or any other segmentation method, that provides as an output representation of anatomical parts.

Then, if possible, and if step 302 is performed, in step 303, the information obtained from DICOM raw 3D scan volume and the autonomous segmentation results of well recognizable neighboring anatomical structures (from step 302) are merged. Combining the information about appearance and classification of neighboring anatomical structures increases the amount of information used for inference in multidimensional autonomous segmentation process by expanding the input data dimensionality. This way the network obtains enhanced information about the data, easing the segmentation of anatomical structures of interest. This can be achieved, for example, by modifying the input data to take the form of color-coded 3D volumes, as shown in Fig. 2C. Alternatively, the process may take place directly inside of the neural network, where separately introduced 3D volume scans (Fig. 2A) and the initial segmentation results (Fig. 2B) can be passed together to a neural network to produce internally the information of higher dimensionality.

Additionally automatically pre-segmentedneighboring structures can also be automatically excluded from the area of interest before the main segmentation process, as they are known to present different anatomical structures, so shouldn't be taken into consideration for the segmentation of anatomical structures of interest.

Next, in step 304, succeeding multidimensional regions of data are determined using predefined parameters, such as the size of the region or the multidimensional stride. The number of regions is dependent on the manually predefined parameters and the data size. The size parameters can be defined in such a way to make the succeeding regions, such as exemplary regions 201, 202 and 203, be determined along the main axis of the data, with overlapping (as shown in Fig. 2D-1) or without overlapping (as shown in Fig. 2D-2), depending on the main axis stride value. To achieve a more complex solution the predefined size of the region can be decreased, inducing multidimensional stride (stride over multiple axes) to analyze the whole dataset. In such a solution, regions of smaller size are determined along multiple axes of the data, with or without overlapping, depending on the predefined stride for each axis. Predefined parameter values are subject to change, based on the application requirements and input data type.

The number of multidimensions depends on the amount of information obtained from convergent sources that are combined before the inference. For example, it is possible to combine a three-dimensional information from medical imaging (DICOM) with another three-dimensional information from automatic segmentation of neighboring structures. This combination produces a four-dimensional input information, but even more dimensions can be added, by providing more information from different sources, for example, information about level identification obtained with a method disclosed in a European patent application EP19169136 by the present applicant, medical imaging information in time domain or any other type of information.

Then, in step 305, if needed, the automatically defined (in step 304) succeeding multidimensional regions (such as 201, 202, and 203) are being subjected to multidimensional resizing, in order to achieve the predefined size. The input information size, for both training the neural network and segmenting anatomical structures of interest (with trained neural network), needs to be the same, so the predefined size is determined by the parameters (from step 105) defining the size of regions used in the neural network training. This ensures input information of the same size for both training the neural network and segmenting anatomical structures of interest with trained neural network.

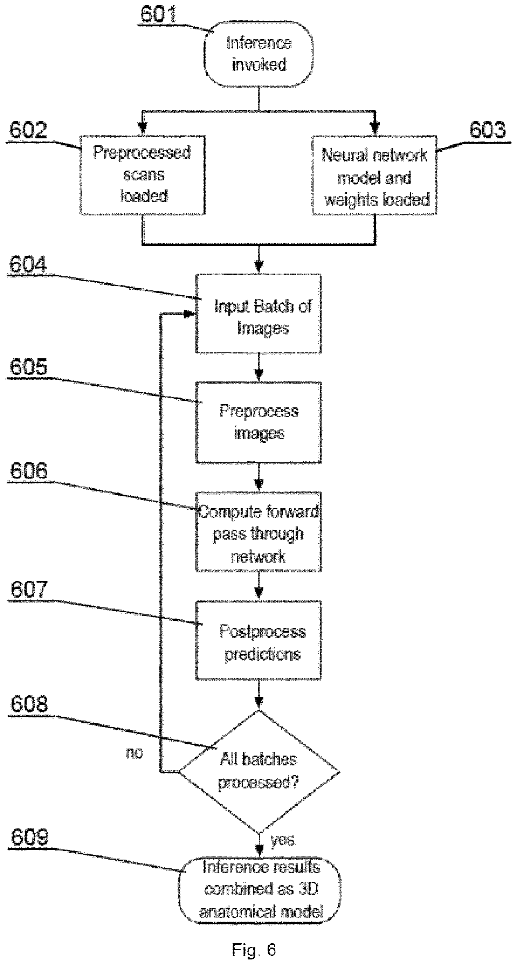

Next, in step 306, the anatomical structures are autonomously segmented by processing the multidimensional regions of data determined in step 304 (or resized regions from step 305), to define the 3D size and shape of the anatomical structures of interest, by means of the pretrained autonomous multidimensional segmentation CNN 400, as shown in Fig. 4, according to the segmentation process presented in Fig. 6.

Then, in step 307, several weak segmentation results (obtained per region) are automatically combined by determining the local overlapping segmentation voxels in order to achieve a strong segmentation result, ensuring proper mapping of anatomical structures and their continuity. The developed method is based on, and resembles, methods widely used in machine learning, called Boosting and Bagging. The developed method is based on the assumption that combining multiple lower quality predictions (referred to in this description as weak segmentation results) for the same voxel, with slightly changed predicting conditions, results in a single high quality prediction (referred to in this description as strong segmentation results), that presents an increased certainty for defining the proper voxel class affiliation. The predictions for voxels contained in the overlapping regions are being automatically recalculated, for example, but not limited to, using mean or median functions for each overlapping voxel separately, or defined groups of voxels.

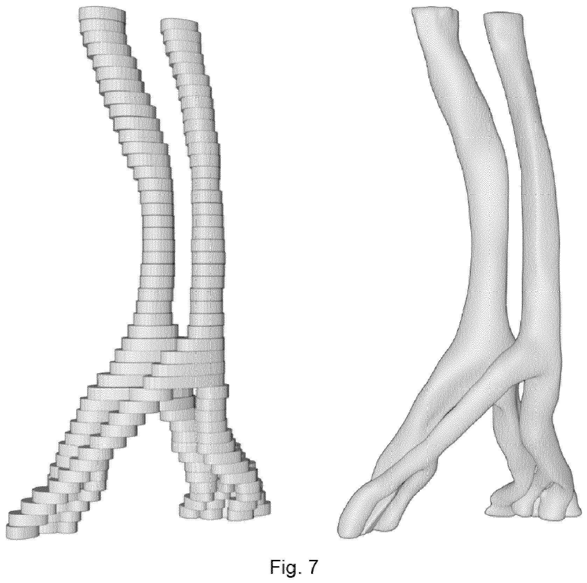

Next, in step 308, raw strong segmentation results are automatically filtered with predefined set of filters and parameters, for enhancing proper shape, location, size and continuity (Fig. 7).

Then, in step 309, the filtered strong segmentation results (from step 308) are automatically analyzed to identify the plurality of classes resembling the anatomical structures of interest.

Finally, in step 310, the identified anatomical structures (309) are visualized. Obtained segmentation results can be combined to a segmented 3D anatomical model. The model can be further converted to a polygonal mesh. The volume and/or mesh representation parameters can be adjusted in terms of change of color, opacity, changing the mesh decimation depending on the needs of the operator.

Fig. 4 shows a convolutional neural network (CNN) architecture 400, hereinafter called the anatomical-structures segmentation CNN, which is utilized in the present method for both semantic and binary segmentation. The network performs voxel-wise class probability mapping using an encoder-decoder architecture, using at least one input as a multidimensional information about appearance (medical imaging radiodensity) and, if needed, the classification of other neighboring anatomical structures in a multidimensional 3D scan volume region. The left side of the network is a contracting path, which includes multidimensional convolution layers 401 and pooling layers 402, and the right side is an expanding path, which includes upsampling or transpose convolution layers 403 and convolutional layers 404 and the output layer 405.

A plurality of multidimensional 3D scan volume regions can be passed to the input layer of the network in order to speed up the training and improve reasoning on the data.

The convolution layers 401 or 404 can be of a standard kind, the dilated kind, or a combination thereof, with ReLU, leaky ReLU or any other activation function attached.

The pooling layers 402 can perform average, max or any other operations on kernels, in order to downsample the data.

The type of upsampling or deconvolution layers 403 can be of a standard kind, the dilated kind, or combination thereof, with ReLU, leaky ReLU or any other activation function attached.

The output layer 405 denotes a softmax or sigmoid stage connected as the network output, preceded by an optional plurality of densely connected hidden layers. Each of these hidden layers can have ReLU, leaky ReLU or any other activation function attached.

The final layer for binary segmentation task recognizes two classes: anatomical structures and the background, while semantic segmentation can be extended to more than two classes, one for each of the anatomical structures of interest.

The encoding-decoding flow is supplemented with additional skipping connections between layers with corresponding sizes (resolutions), which improves the network performance through information merging across different prediction stages. It enables either the use of max-pooling indices from the corresponding encoder stage to downsample, or learning the deconvolution filters to upsample.

The general CNN architecture can be adapted to consider regions of different dimensions. The number of layers and number of filters within a layer are also subject to change, depending on application requirements and anatomical areas to be segmented.

Additionally Select-Attend-Transfer (SAT) gates or Generative Adversarial Networks (GAN) can be used to increase the final quality of the segmentation. Introducing Select-Attend-Transfer gates to the encoder-decoder neural network results in focusing the network on the most important anatomical structure features and their localization, simultaneously decreasing the memory consumption. Moreover, the Generative Adversarial Networks can be used to produce new artificial training examples.

The semantic segmentation can classify multiple classes, each representing anatomical structures or their parts of a different kind. For example, the vascular structures may include aorta, vena cava, and other circulatory system vessels; spine and its parts, such as vertebral body 16, pedicles 15, transverse processes 14, lamina 13 and/or spinous process 11; nerves may include upper and lower extremities, cervical, thoracic or lumbar plexus, the spinal cord, nerves of the peripheral nervous system (e.g., sciatic nerve, median nerve, brachial plexus), cranial nerves; and other structures, such as muscles, ligaments, intervertebral discs, joints, cerebrospinal fluid.

Fig. 5 shows a flowchart of a training process, which can be used to train the anatomical-structures segmentation CNN 400 shown in Fig. 4. The objective of the training for the segmentation CNN 400 is to tune the internal parameters of the network, so it is able to recognize and segment a multidimensional 3D scan volume region. The training database may be split into a plurality of subsets, such as, a training set used to train the model, a validation set used to quantify the quality of the model, and a test set used to confirm the network robustness.

The training starts at 501. At 502, batches of training multidimensional regions are read from the training set, one batch at a time. For the segmentation, multidimensional regions represent the input of the CNN, and the corresponding pre-segmented 3D volumes, which were manually segmented by a human, represent its desired output.

At 503 the original 3D images (ROIs) can be augmented. Data augmentation is performed on these 3D images (ROIs) to make the training set more diverse. The input and output pair of three-dimensional images (ROIs) is subjected to the same combination of transformations.

At 504, the original 3D images (ROIs) and the augmented 3D images (ROIs) are then passed through the layers of the CNN in a standard, forward pass. The forward pass returns the results, which are then used to calculate at 505 the value of the loss function (i.e., the difference between the desired output and the output computed by the CNN). The difference can be expressed using a similarity metric (e.g., mean squared error, mean average error, categorical cross-entropy, or another metric).

At 506, weights are updated as per the specified optimizer and optimizer learning rate. The loss may be calculated, for example, using a per-pixel cross-entropy loss function and the Adam update rule.

The loss is also back propagated through the network, and the gradients are computed. Based on the gradient values, the network weights are updated. The process, beginning with the 3D images (ROIs) batch read, is repeated continuously until the end of the training session is reached at 506.

Then, at 508, the performance metrics are calculated using a validation dataset - which is not explicitly used in training set. This is done in order to check at 509 whether not the model has improved. If it is not the case, the early stop counter is incremented by one at 514, if its value has not reached a predefined maximum number of epochs at 515. The training process continues until there is no further improvement obtained at 516. Then the model is saved at 510 for further use, and the early stop counter is reset at 511. As the final step in a session, learning rate scheduling can be applied. The session at which the rate is to be changed are predefined. Once one of the session numbers is reached at 512, the learning rate is set to one associated with this specific session number at 513.

Once the training process is complete, the network can be used for inference (i.e., utilizing a trained model for autonomous segmentation of new medical images).

Fig. 6 shows a flowchart of an inference process for the anatomical-structures segmentation CNN 400.

After inference is invoked at 601, a set of scans (three dimensional images) are loaded at 602 and the segmentation CNN 400 and its weights are loaded at 603.

At 604, one batch of three-dimensional images (ROIs) at a time is processed by the inference server.

At 605, the images are preprocessed (e.g., normalized, cropped, etc.) using the same parameters that were utilized during training. In at least some implementations, inference-time distortions are applied, and the average inference result is taken on, for example, 10 distorted copies of each input 3D image (ROI). This feature creates inference results that are robust to small variations in brightness, contrast, orientation, etc.

At 606, a forward pass through the segmentation CNN 400 is computed.

At 607, the system may perform post-processing such as linear filtering (e.g., Gaussian filtering), or nonlinear filtering ( e.g., median filtering, and morphological opening or closing).

At 608, if not all batches have been processed, a new batch is added to the processing pipeline until inference has been performed at all input 3D images (ROIs).

Finally, at 609, the inference results are saved and can be combined to a segmented 3D anatomical model. The model can be further converted to a polygonal mesh for the purpose of visualization. The volume and/or mesh representation parameters can be adjusted in terms of change of color, opacity, changing the mesh decimation depending on the needs of the operator.

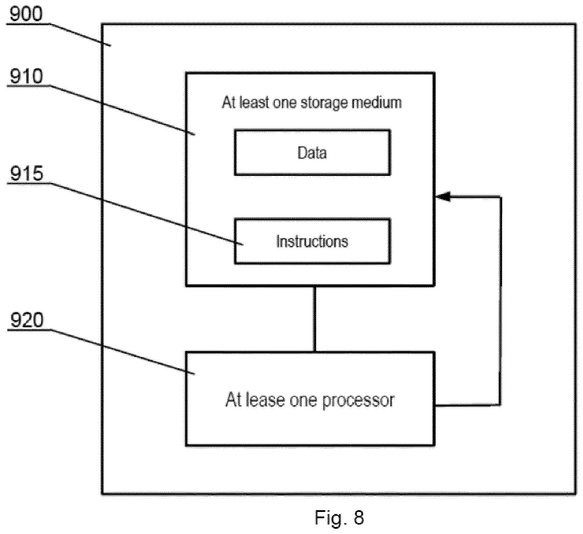

The functionality described herein can be implemented in a computer-implemented system 900, such as shown in Fig. 8. The system may include at least one non-transitory processor-readable storage medium that stores at least one of processor-executable instructions or data and at least one processor communicably coupled to at least one non-transitory processor-readable storage medium. At least one processor is configured to perform the steps of the methods presented herein.

The computer-implemented system 900, for example a machine-learning system, may include at least one non-transitory processor-readable storage medium 910 that stores at least one of processor-executable instructions 915 or data; and at least one processor 920 communicably coupled to the at least one non-transitory processor-readable storage medium 910. At least one processor 920 may be configured to (by executing the instructions 915) to perform the steps of the method of Fig. 3.

## Claims

1. A method for autonomous multidimensional segmentation of anatomical structures from three-dimensional, 3D, scan volumes, the method comprising the following steps:
- receiving (301) input data including the 3D scan volumes comprising a set of medical scan images comprising the anatomical structures;
- automatically defining (304) succeeding multidimensional regions of the input data;
- autonomously processing (306), by means of a pre-trained segmentation convolutional neural network, CNN, (400), the defined multidimensional regions to determine weak segmentation results that define a 3D shape, location, and size of the anatomical structures;
- automatically combining (307) multiple weak segmentation results by determining voxels within the defined multidimensional regions that overlap and combining the weak segmentation results determined for the same voxel, to obtain raw strong segmentation results for the voxels with improved accuracy;
- autonomously filtering (308) the raw strong segmentation results with a predefined set of filters and parameters for enhancing the 3D shape, location, and size of the anatomical structures to obtain filtered strong segmentation results; and
- autonomously identifying (309) a plurality of classes of the anatomical structures from the filtered strong segmentation results;

2. The method according to claim 1, further comprising, after receiving (301) the 3D scan volume:
- autonomously processing (302) the 3D scan volume to perform a semantic and/or binary segmentation of neighboring anatomical structures, in order to obtain autonomous segmentation results defining a 3D representation of the neighboring anatomical structures; and
- increasing a dimensionality of the input data by combining (303) the autonomous segmentation results for the neighboring structures with information obtained from the 3D scan volumes, the multidimensional regions of the input data being defined (304) after the dimensionality of the input data has been increased.

3. The method according to any one of the previous claims, further comprising visualizing (310) an output including the anatomical structures after autonomously identifying (309) the plurality of classes of the anatomical structures.

4. The method according to any one of the previous claims, wherein the segmentation CNN (400) is a fully convolutional neural network model with or without layer skip connections.

5. The method according to claim 4, wherein the segmentation CNN (400) includes a contracting path and an expanding path.

6. The method according to claim 5, wherein the segmentation CNN (400) comprises, in the contracting path, a number of convolutional layers and a number of pooling layers, where each pooling layer is preceded by at least one convolutional layer.

7. The method according to claim 5 or 6, wherein the segmentation CNN (400) comprises, in the expanding path, a number of convolutional layers and a number of upsampling or deconvolutional layers, where each upsampling or deconvolutional layer is preceded by at least one convolutional layer.

8. The method according to claim 4, wherein an output of the segmentation CNN (400) is improved by Select-Attend-Transfer, SAT, gates.

9. The method according to claim 4, wherein an output of the segmentation CNN (400) is improved by Generative Adversarial Networks, GAN.

10. The method according to any one of the previous claims, wherein the set of medical scan images is collected from an intraoperative scanner.

11. The method according to any one of the previous claims, wherein the set of medical scan images is collected from a presurgical stationary scanner.

12. The method according to claim 3, further comprising combining the filtered strong segmentation results into a 3D anatomical model and converting the 3D anatomical model into a polygonal mesh for visualizing (310) the output.

13. The method according to any one of the previous claims, further comprising, before autonomously processing (306) the defined multidimensional regions, performing resizing (305) of the defined multidimensional regions in order to achieve a predefined size for each multidimensional region for inputting into the pre-trained segmentation CNN,
the pre-trained segmentation CNN trained using training data including multidimensional regions having the predefined size.

14. The method of according to any one of the preceding claims, wherein the succeeding multidimensional regions are defined (304) based on predefined parameters including a size of each multidimensional region and a multidimensional stride.

15. A computer-implemented system, comprising:
- at least one non-transitory processor-readable storage medium (910) that stores at least one processor-executable instruction (915) or data; and
- at least one processor (920) communicably coupled to at least one non-transitory processor-readable storage medium (910), wherein the at least one processor (920) is configured to perform the steps of the method of any one of the previous claims.

## Patentansprüche

1. Verfahren zur autonomen mehrdimensionalen Segmentierung anatomischer Strukturen aus dreidimensionalen, 3D, Scan-Volumina, das Verfahren umfassend die folgenden Schritte:
- Empfangen (301) von Eingangsdaten, die die 3D-Scan-Volumina beinhalten, umfassend einen Satz medizinischer Scan-Bilder, umfassend die anatomischen Strukturen;
- automatisches Definieren (304) aufeinanderfolgender mehrdimensionaler Bereiche der Eingangsdaten;
- autonomes Verarbeiten (306), mittels eines vortrainierten Segmentierung neuronalen faltenden Netzwerks, CNN, (400), der definierten mehrdimensionalen Bereiche, um schwache Segmentierungsresultate zu bestimmen, die eine 3D-Form, Position und Größe der anatomischen Strukturen definieren;
- automatisches Kombinieren (307) mehrerer schwacher Segmentierungsresultate durch Bestimmen von Voxeln innerhalb der definierten mehrdimensionalen Bereiche, die sich überlappen, und Kombinieren der schwachen Segmentierungsresultate, die für denselben Voxel bestimmt wurden, um rohe starke Segmentierungsresultate für die Voxel mit verbesserter Genauigkeit zu erlangen;
- autonomes Filtern (308) der rohen starken Segmentierungsresultate mit einem vordefinierten Satz von Filtern und Parametern zum Verbessern der 3D-Form, Position und Größe der anatomischen Strukturen, um gefilterte starke Segmentierungsresultate zu erlangen; und
- autonomes Identifizieren (309) einer Vielzahl von Klassen der anatomischen Strukturen aus den gefilterten starken Segmentierungsresultaten.

2. Verfahren nach Anspruch 1, ferner umfassend, nach Empfangen (301) des 3D-Scan-Volumens:
- autonomes Verarbeiten (302) des 3D-Scan-Volumens, um eine semantische und/oder binäre Segmentierung benachbarter anatomischer Strukturen auszuführen, um autonome Segmentierungsresultate zu erlangen, die eine 3D-Darstellung der benachbarten anatomischen Strukturen definieren; und
- Erhöhen einer Dimensionalität der Eingangsdaten durch Kombinieren (303) der autonomen Segmentierungsresultate für die benachbarten Strukturen mit Informationen, die aus den 3D-Scan-Volumina erlangt werden, wobei die mehrdimensionalen Bereiche der Eingangsdaten definiert werden (304), nachdem die Dimensionalität der Eingangsdaten erhöht wurde.

3. Verfahren nach einem der vorherigen Ansprüche, ferner umfassend ein Visualisieren (310) eines Ausgangs, der die anatomischen Strukturen beinhaltet, nach autonomem Identifizieren (309) der Vielzahl von Klassen der anatomischen Strukturen.

4. Verfahren nach einem der vorherigen Ansprüche, wobei das Segmentierungs-CNN (400) ein vollständig neuronales faltendes Netzwerkmodell mit oder ohne Schicht-Sprungverbindungen ist.

5. Verfahren nach Anspruch 4, wobei das Segmentierungs-CNN (400) einen kontrahierenden Pfad und einen expandierenden Pfad beinhaltet.

6. Verfahren nach Anspruch 5, wobei das Segmentierungs-CNN (400) in dem kontrahierenden Pfad eine Anzahl von Filterschichten und eine Anzahl von Aggregationsschichten umfasst, wobei jeder Aggregationsschicht mindestens eine Filterschicht vorausgeht.

7. Verfahren nach Anspruch 5 oder 6, wobei das Segmentierungs-CNN (400) in dem expandierenden Pfad eine Anzahl von Filterschichten und eine Anzahl von Expansions- oder Dekonvolutionsschichten umfasst, wobei jeder Expansions- oder Dekonvolutionsschicht mindestens eine Filterschicht vorausgeht.

8. Verfahren nach Anspruch 4, wobei ein Ausgang des Segmentierungs-CNN (400) durch Gates für Select-Attend-Transfer, SAT, verbessert wird.

9. Verfahren nach Anspruch 4, wobei ein Ausgang des Segmentierungs-CNN (400) durch Generative Adversarial Networks, GAN, verbessert wird.

10. Verfahren nach einem der vorherigen Ansprüche, wobei der Satz medizinischer Scanbilder von einem intraoperativen Scanner gesammelt wird.

11. Verfahren nach einem der vorherigen Ansprüche, wobei der Satz medizinischer Scan-Bilder von einem präoperativen stationären Scanner gesammelt wird.

12. Verfahren nach Anspruch 3, ferner umfassend ein Kombinieren der gefilterten starken Segmentierungsresultate zu einem anatomischen 3D-Modell und Umwandeln des anatomischen 3D-Modells in ein polygonales Netz zum Visualisieren (310) des Ausgangs.

13. Verfahren nach einem der vorherigen Ansprüche, ferner umfassend, dass vor einem autonomen Verarbeiten (306) der definierten mehrdimensionalen Bereiche eine Größenänderung (305) der definierten mehrdimensionalen Bereiche ausgeführt wird, um eine vordefinierte Größe für jeden mehrdimensionalen Bereich zum Eingang in das vortrainierte Segmentierungs-CNN zu erreichen,
wobei das vortrainierte Segmentierungs-CNN unter Verwendung von Trainingsdaten trainiert wird, die mehrdimensionale Bereiche mit der vordefinierten Größe aufweisen.

14. Verfahren nach einem der vorherigen Ansprüche, wobei die aufeinanderfolgenden mehrdimensionalen Bereiche basierend auf vordefinierten Parametern, einschließlich einer Größe von jedem mehrdimensionalen Bereich und einer mehrdimensionalen Schrittweite, definiert (304) sind.

15. Computerimplementiertes System, umfassend:
- mindestens ein nicht-transitorisches, prozessorlesbares Speichermedium (910), das mindestens eine prozessorausführbare Anweisung (915) oder Daten speichert; und
- mindestens einen Prozessor (920), der kommunikativ mit mindestens einem nicht-transitorischen, prozessorlesbaren Speichermedium (910) gekoppelt ist, wobei der mindestens eine Prozessor (920) konfiguriert ist, um die Schritte des Verfahrens nach einem der vorherigen Ansprüche durchzuführen.

## Revendications

1. Méthode de segmentation multidimensionnelle autonome de structures anatomiques à partir de volumes de balayage tridimensionnels, 3D, le procédé comprenant les étapes suivantes :
- la réception (301) de données d'entrée comportant les volumes de balayage 3D comprenant un ensemble d'images médicales de balayage comprenant les structures anatomiques ;
- la définition automatique (304) de zones multidimensionnelles successives des données d'entrée ;
- le traitement autonome (306), au moyen d'un réseau neuronal convolutif, CNN, de segmentation pré-entraîné (400), des zones multidimensionnelles définies pour déterminer des résultats de segmentation faibles qui définissent la forme, l'emplacement et la taille 3D des structures anatomiques ;
- la combinaison automatique (307) de multiples résultats de segmentation faibles en déterminant des voxels dans les zones multidimensionnelles définies qui se chevauchent et en combinant les résultats de segmentation faibles déterminés pour le même voxel, pour obtenir des résultats de segmentation forts bruts pour les voxels avec une précision améliorée ;
- le filtrage autonome (308) des résultats de segmentation forts bruts avec un ensemble prédéfini de filtres et de paramètres pour améliorer la forme, l'emplacement et la taille 3D des structures anatomiques afin d'obtenir des résultats de segmentation forts filtrés ; et
- l'identification autonome (309) d'une pluralité de classes des structures anatomiques à partir des résultats de segmentation forts filtrés.

2. Procédé selon la revendication 1, comprenant en outre, après la réception (301) du volume de balayage 3D :
- le traitement autonome (302) du volume de balayage 3D pour effectuer une segmentation sémantique et/ou binaire des structures anatomiques voisines, afin d'obtenir des résultats de segmentation autonomes définissant une représentation 3D des structures anatomiques voisines ; et
- l'augmentation d'une dimensionnalité des données d'entrée par la combinaison (303) des résultats de segmentation autonome pour les structures voisines avec des informations obtenues à partir des volumes de balayage 3D, les zones multidimensionnelles des données d'entrée étant définies (304) après l'augmentation de la dimensionnalité des données d'entrée.

3. Procédé selon l'une quelconque des revendications précédentes, comprenant en outre la visualisation (310) d'un résultat comportant les structures anatomiques après l'identification autonome (309) de la pluralité de classes des structures anatomiques.

4. Procédé selon l'une quelconque des revendications précédentes, ledit CNN de segmentation (400) étant un modèle de réseau neuronal entièrement convolutif avec ou sans connexions par saut de couche.

5. Procédé selon la revendication 4, ledit CNN de segmentation (400) comprenant un chemin de contraction et un chemin d'expansion.

6. Procédé selon la revendication 5, ledit CNN de segmentation (400) comprenant, dans le chemin de contraction, un certain nombre de couches de convolution et un certain nombre de couches de regroupement, chaque couche de regroupement étant précédée d'au moins une couche de convolution.

7. Procédé selon la revendication 5 ou 6, ledit CNN de segmentation (400) comprenant, dans le trajet d'expansion, un certain nombre de couches de convolution et un certain nombre de couches de suréchantillonnage ou de déconvolution, chaque couche de suréchantillonnage ou de déconvolution étant précédée d'au moins une couche de convolution.

8. Procédé selon la revendication 4, le résultat dudit CNN de segmentation (400) étant amélioré par des portes Sélectionner-Attendre-Transférer, SAT.

9. Procédé selon la revendication 4, le résultat dudit CNN de segmentation (400) étant amélioré par des réseaux antagonistes génératifs, GAN.

10. Procédé selon l'une quelconque des revendications précédentes, ledit ensemble d'images médicales de balayage reçues étant collecté à partir d'un scanner peropératoire.

11. Procédé selon l'une quelconque des revendications précédentes, ledit ensemble d'images médicales de balayage étant collecté à partir d'un scanner stationnaire pré-chirurgical.

12. Procédé selon la revendication 3, comprenant en outre la combinaison des résultats de segmentation forts filtrés en un modèle anatomique 3D et la conversion du modèle anatomique 3D en un maillage polygonal permettant la visualisation (310) du résultat.

13. Procédé selon l'une quelconque des revendications précédentes, comprenant en outre, avant le traitement autonome (306) des zones multidimensionnelles définies, l'exécution d'un redimensionnement (305) des zones multidimensionnelles définies afin d'obtenir une taille prédéfinie pour chaque zone multidimensionnelle pour l'entrée dans le CNN de segmentation pré-entraîné,
le CNN de segmentation pré-entraîné étant entraîné à l'aide de données d'apprentissage comportant des zones multidimensionnelles présentant la taille prédéfinie.

14. Procédé selon l'une quelconque des revendications précédentes, lesdites zones multidimensionnelles successives étant définies (304) sur la base de paramètres prédéfinis comportant la taille de chaque zone multidimensionnelle et le pas multidimensionnel.

15. Système mis en oeuvre par ordinateur comprenant :
- au moins un support de stockage non transitoire lisible par processeur (910) qui stocke au moins une instruction exécutable par processeur (915) ou donnée ; et
- au moins un processeur (920) couplé en communication à au moins un support de stockage non transitoire lisible par processeur (910), au moins un processeur (920) étant configuré pour exécuter les étapes du procédé selon l'une quelconque des revendications précédentes.
